# EUROPÄISCHE PATENTSCHRIFT

(11) **EP 1 902 009 B1**
(45) Veröffentlichungstag und Bekanntmachung des Hinweises auf die Patenterteilung: **28.07.2010**
(21) Anmeldenummer: 06763634.0
(22) Anmeldetag: 12.06.2006
(51) Int. Cl.: C07C 41/50, C07C 43/30

(54) **VERFAHREN ZUR HERSTELLUNG VON POLYOXYMETHYLENDIALKYLETHERN AUS TRIOXAN UND DIALKYLETHER**
METHOD FOR THE PRODUCTION OF POLYOXYMETHYLENE DIALKYL ETHERS FROM TRIOXAN AND DIALKYLETHERS
PROCEDE DE PRODUCTION DE POLYOXYMETHYLENE-DIALKYLETHERS A PARTIR DE TRIOXANE ET DE DIALKYLETHER

(30) Priorität: 15.06.2005 DE 102005027690
(43) Veröffentlichungstag der Anmeldung: 26.03.2008
(73) Patentinhaber: BASF SE, 67056 Ludwigshafen (DE)
(72) Erfinder: STRÖFER, Eckhard, 68163 Mannheim (DE); SCHELLING, Heiner, 67281 Kirchheim (DE); HASSE, Hans, 67661 Kaiserslautern (DE); BLAGOV, Sergej, 70195 Stuttgart (DE)
(86) Internationale Anmeldenummer: PCT/EP2006/063074
(87) Internationale Veröffentlichungsnummer: WO 2006/134081

(56) Entgegenhaltungen:
- US-B1- 6 392 102

## Beschreibung

Die Erfindung betrifft ein Verfahren zur Herstellung von Polyoxymethylendialkylethern.

Polyoxymethylendimethylether stellen eine homologe Reihe der allgemeinen Formel

CH₃O(CH₂O)ₙCH₃

dar, worin n eine Zahl ≥ 1 bedeutet. Wie das Stamm-Molekül der homologen Reihe Methylal CH₃O(CH₂O)CH₃ (n = 1) sind die Polyoxymethylendimethylether Acetale. Sie werden durch Reaktion von Methanol mit wässrigem Formaldehyd in Gegenwart eines sauren Katalysators hergestellt. Wie andere Acetale sind sie unter neutralen oder alkalischen Bedingungen stabil, werden aber schon von verdünnten Säuren angegriffen. Durch Hydrolyse werden sie dabei in einem ersten Schritt zu Halbacetalen und Methanol umgesetzt. In einem zweiten Schritt werden die Halbacetale zu Formaldehyd und Methanol hydrolysiert.

Im Labormaßstab werden Polyoxymethylendimethylether durch Erhitzen von Polyoxymethylenglykol oder Paraformaldehyd mit Methanol in Gegenwart von Spuren von Schwefelsäure oder Salzsäure bei Temperaturen von 150 bis 180 °C und Reaktionszeiten von 12 bis 15 Stunden hergestellt. Dabei kommt es zu Zersetzungsreaktionen unter Bildung von Kohlendioxid und zur Bildung von Dimethylether. Bei einem Paraformaldehyd oder Polyoxymethylenglykol : Methanol-Verhältnis von 6 : 1 werden Polymere mit n > 100, im Allgemeinen n = 300 - 500 erhalten. Die Produkte werden mit Natriumsulfitlösung gewaschen und anschließend durch fraktionierte Kristallisation fraktioniert.

US 2,449,469 beschreibt ein Verfahren, bei dem Methylal mit Paraformaldehyd oder einer konzentrierten Formaldehyd-Lösung in Gegenwart von Schwefelsäure erhitzt wird. Dabei werden Polyoxymethylendimethylether mit 2 bis 4 Formaldehyd-Einheiten pro Molekül erhalten.

In jüngerer Zeit haben Polyoxymethylendimethylether als Dieselkraftstoff-Additive Bedeutung erlangt. Zur Verringerung der Rauch- und Russbildung bei der Verbrennung von herkömmlichem Dieselkraftstoff werden diesem sauerstoffhaltige Verbindungen, welche nur wenige oder überhaupt keine C-C-Bindungen aufweisen, wie beispielsweise Methanol, zugesetzt. Jedoch sind derartige Verbindungen häufig unlöslich in Dieselkraftstoff und setzen die Cetan-Zahl und/oder den Flammpunkt des Dieselkraftstoffgemisches herab.

US 5,746,785 beschreibt die Herstellung von Polyoxymethylendimethylethern mit einem Molgewicht von 80 bis 350, entsprechend n = 1 - 10, durch Reaktion von 1 Teil Methylal mit 5 Teilen Paraformaldehyd in Gegenwart von 0,1 Gew.-% Ameisensäure bei einer Temperatur von 150 bis 240 °C beziehungsweise durch Reaktion von 1 Teil Methanol mit 3 Teilen Paraformaldehyd bei einer Temperatur von 150 bis 240 °C. Die erhaltenen Polyoxymethylendimethylether werden in Mengen von 5 bis 30 Gew.-% einem Dieselkraftstoff zugesetzt.

US 6,392,102 beschreibt die Herstellung von Polyoxymethylendimethylethern durch Umsetzung eines Einsatzstroms enthaltend Methanol und Formaldehyd, welcher durch Oxidation von Dimethylether erhalten wurde, in Gegenwart eines sauren Katalysators und gleichzeitiger Auftrennung der Reaktionsprodukte in einer katalytischen Destillationskolonne. Dabei werden Methylal, Methanol, Wasser und Polyoxymethylendimethylether erhalten.

EP-A 1 070 755 offenbart die Herstellung von Polyoxymethylendimethylethern mit 2 bis 6 Formaldehyd-Einheiten im Molekül durch Umsetzung von Methylal mit Paraformaldehyd in Gegenwart von Trifluorsulfonsäure. Dabei werden mit einer Selektivität von 94,8 % Polyoxymethylendimethylether mit n = 2 - 5 gebildet, wobei zu 49,6 % das Dimer (n = 2) erhalten wird. Die erhaltenen Polyoxymethylendimethylether werden in Mengen von 4 bis 11 Gew.-% einem Dieselkraftstoff zugesetzt.

Nachteilig an den bekannten Verfahren zur Herstellung der niederen Polyoxymethylendimethylether (mit n = 1-10) ist, dass ganz überwiegend das Dimer erhalten wird. Nachteilig an den Verfahren, die von Formaldehyd und Methanol ausgehen, ist zudem, dass als Reaktionsprodukt Wasser gebildet wird, welches in Gegenwart der sauren Katalysatoren bereits gebildete Polyoxymethylendimethylether hydrolysiert. Dabei werden instabile Halbacetale gebildet. Die instabilen Halbacetale setzen den Flammpunkt des Dieselkraftstoffgemischs herab und beeinträchtigen somit dessen Qualität. Ein zu niedriger Flammpunkt des Dieselkraftstoffgemischs führt aber dazu, dass die durch einschlägige DIN-Normen vorgegebenen Spezifikationen nicht mehr erfüllt werden. Halbacetale sind aber wegen vergleichbarer Siedepunkte schwer von Polyoxymethylendimethylethern abzutrennen. Das als Hauptprodukt gebildete Dimer weist einen niedrigen Siedepunkt auf und setzt damit ebenfalls den Flammpunkt herab, wodurch es als Dieselkraftstoff-Additiv weniger geeignet ist.

Aufgabe der Erfindung ist es, ein verbessertes Verfahren zur Herstellung von Polyoxymethylendialkylethern bereitzustellen, welches die Nachteile des Standes der Technik nicht aufweist. Aufgabe der Erfindung ist es insbesondere, ein Verfahren zur Herstellung von Polyoxymethylendialkylethern bereitzustellen, welche als Dieselkraftstoff-Additive besonders gut geeignet sind. Besonders gut geeignet sind die Polyoxymethylendimethylether und Polyoxymethylendiethylether mit n = 3 und 4 (Trimer, Tetramer). Aufgabe der Erfindung ist es insbesondere, ein Verfahren zur Herstellung von Polyoxymethylendialkylethern mit einem besonders hohen Anteil an Trimer bereitzustellen.

Gelöst wird die Aufgabe durch ein Verfahren zur Herstellung von Polyoxymethylendialkylethern der Formel

H₂ₘ₊₁<CₘO(CH₂O)ₙCₘH₂ₘ₊₁

mit n=2-10,
m, gleich oder verschieden, = 1 oder 2,
bei dem ein Dialkylether, ausgewählt aus Dimethylether (DME), Methylethylether und Diethylether (DEE), und Trioxan in einen Reaktor eingespeist werden und in Gegenwart eines sauren Katalysators umgesetzt werden, wobei die durch den Dialkylether, Trioxan und/oder den Katalysator in das Reaktionsgemisch eingebrachte Wassermenge < 1 Gew.-%, bezogen auf das Reaktionsgemisch, beträgt.

Es können auch Gemische der Dialkylether eingesetzt werden. Bevorzugt wird die Umsetzung mit DME durchgeführt.

Durch den Einsatz von Dialkylether als Edukt kann die Reaktion weitgehend wasserfrei geführt werden kann, da Wasser nicht als Nebenprodukt entsteht, wie nachstehender Reaktionsgleichung zu entnehmen ist:

CH₃OCH₃ + (CH₂O)₃ → CH₃O - (CH₂O)₃ - CH₃

In Gegenwart von Wasser oder Alkoholen würden chemische Reaktionen ablaufen, die zu einer Vielzahl von Poly(oxymethylen)glykolen und Hemiformalen führen würden. Es würden reaktive Azeotrope gebildet, die zu einem komplexen Phasenverhalten beim Versuch der destillativen Trennung führen würden.

Die Umsetzung von Dialkylether und Trioxan wird im Allgemeinen bei einer Temperatur von -20 bis +200 °C, vorzugsweise 0 bis 150 °C, und einem Druck von 1 bis 200 bar, vorzugsweise 2 bis 100 bar durchgeführt. Das Molverhältnis Dialkylether : Trioxan beträgt im Allgemeinen 0,1 bis 10, vorzugsweise 0,5 bis 5.

Der saure Katalysator kann ein homogener oder heterogener saurer Katalysator sein. Geeignete saure Katalysatoren sind Mineralsäuren wie weitgehend wasserfreie Schwefelsäure, Sulfonsäuren wie Trifluormethansulfonsäure und para-Toluolsulfonsäure, Heteropolysäuren, saure Ionenaustauscherharze, Zeolithe, Aluminosilikate, Siliciumdioxid, Aluminiumoxid, Titandioxid und Zirkondioxid. Oxidische Katalysatoren können, um deren Säurestärke zu erhöhen, mit Sulfat- oder Phosphat-Gruppen dotiert sein, im Allgemeinen in Mengen von 0,05 bis 10 Gew.-%. Die Umsetzung kann in einem Rührkesselreaktor (CSTR) oder einem Rohrreaktor durchgeführt werden. Wird ein heterogener Katalysator eingesetzt, ist ein Festbettreaktor bevorzugt. Wird ein Katalysator-Festbett verwendet, kann das Produktgemisch anschließend mit einem Anionenaustauscherharz in Kontakt gebracht werden, um ein im Wesentlichen säurefreies Produktgemisch zu erhalten.

Die durch Dialkylether und Trioxan sowie durch den Katalysator eingebrachte Wassermenge beträgt insgesamt < 1 Gew.-%, bevorzugt < 0,5 Gew.%, besonders bevorzugt < 0,2 Gew.-% und insbesondere < 0,1 Gew.-%, bezogen auf das Reaktionsgemisch aus Dialkylether, Trioxan und den Katalysator. Hierzu werden praktisch wasserfreies Trioxan und Dialkylether eingesetzt und die gegebenenfalls durch den Katalysator eingebrachte Wassermenge begrenzt. Die in Gegenwart von Wasser aus bereits gebildetem Polyoxymethylendialkylether durch Hydrolyse gebildeten Halbacetale (Monoether) beziehungsweise Polyoxymethylenglykole weisen einen vergleichbaren Siedepunkt wie die Polyoxymethylendialkylether auf, wodurch eine Abtrennung der Polyoxymethylendialkylether von diesen Nebenprodukten erschwert wird.

Um gezielt Polyoxymethylendialkylether mit n = 3 und n = 4 (Trimer, Tetramer) zu erhalten, wird aus dem Produktgemisch der Umsetzung von Dialkylether mit Trioxan eine Fraktion enthaltend das Trimere und Tetramere abgetrennt und werden nicht umgesetzter Dialkylether, Trioxan und Polyoxymethylendialkylether mit n < 3 in die sauer katalysierte Umsetzung zurückgeführt. In einer weiteren Ausführungsform des erfindungsgemäßen Verfahrens werden zusätzlich auch die Polyoxymethylendialkylether mit n > 4 in die Umsetzung zurückgeführt. Durch die Rückführung wird besonders viel Trimer erhalten.

In einer besonders bevorzugten Ausführungsform wird aus dem Produktgemisch der sauer katalysierten Umsetzung von Dialkylether, bevorzugt DME, mit Trioxan eine erste Fraktion enthaltend den Dialkylether, bevorzugt DME, eine zweite Fraktion enthaltend das Dimer (n = 2) und Trioxan, eine dritte Fraktion enthaltend das Trimere und Tetramere (n = 3, 4) und eine vierte Fraktion enthaltend das Pentamere und höhere Homologe (n > 4) gewonnen. Hierbei ist es insbesondere bevorzugt, die Auftrennung des Produktgemischs der sauer katalysierten Umsetzung von Dialkylether mit Trioxan in drei hintereinander geschalteten Destillationskolonnen durchzuführen, wobei die erste Fraktion in einer Destillationskolonne oder einem Verdampfer von dem Produktgemisch der Umsetzung abgetrennt wird, aus dem verbleibenden Gemisch in einer zweiten Destillationskolonne die zweite Fraktion abgetrennt wird und das verbleibende Gemisch in einer dritten Destillationskolonne in die dritte und die vierte Fraktion aufgetrennt wird. Hierbei kann die erste Destillationskolonne bzw. der Verdampfer beispielsweise bei einem Druck von 0,1 bis 100 bar, die zweite Destillationskolonne beispielsweise bei einem Druck von 0,05 bis 1 bar und die dritte Destillationskolonne beispielsweise bei einem Druck von 0,001 bis 0,5 bar betrieben werden. Vorzugsweise werden die erste und die zweite Fraktion, besonders bevorzugt zusätzlich auch die vierte Fraktion in die Umsetzung zurückgeführt.

Wird ein homogener Katalysator, beispielsweise eine Mineralsäure oder eine Sulfonsäure eingesetzt, so verbleibt dieser in der vierten Fraktion und wird mit dieser in die sauer katalysierte Umsetzung zurückgeführt.

Die Erfindung wird nachstehend unter Bezugnahme auf die Zeichnung näher erläutert.

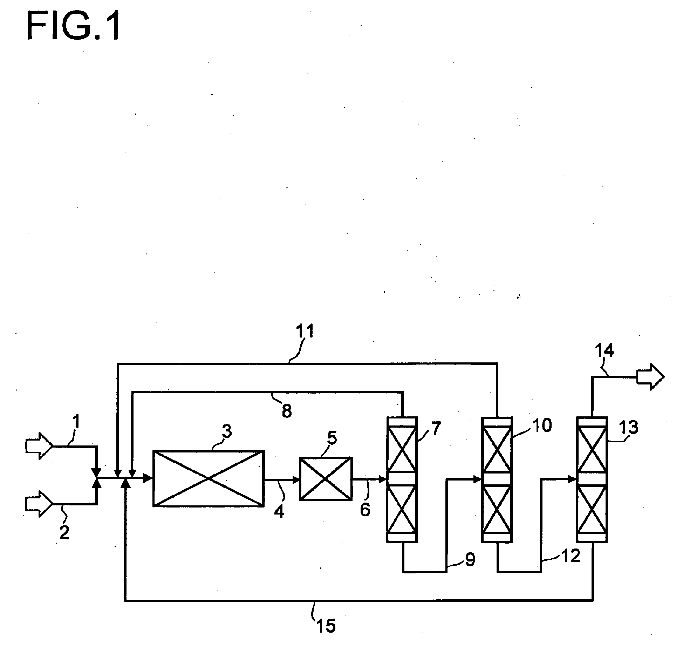

Figur 1 gibt ein Verfahrensfließbild gemäß einer Ausführungsform des erfindungsgemäßen Verfahrens wieder.

Ein Einsatzstrom **1** aus Dialkylether und ein Einsatzstrom **2** aus Trioxan werden gemeinsam mit den Rückführströmen **8, 11** und **15** in den Reaktor **3** eingespeist und dort in Gegenwart eines heterogenen sauren Katalysators zu dem Produktgemisch **4** umgesetzt, welches Dialkylether, Trioxan und Polyoxymethylendialkylether mit n = 2 bis 10 enthält. Der Produktstrom **4** wird durch ein Bett **5** aus Anionenaustauscherharz geleitet, wobei ein im Wesentlichen säurefreies Produktgemisch **6** erhalten wird. Dieses wird in eine erste Destillationskolonne **7** eingespeist, in der Dialkylether als Rückführstrom **8** über Kopf abgetrennt wird. Der Sumpfabzug **9** der ersten Kolonne **7** wird in eine zweite Destillationskolonne **10** eingeleitet, in der das Dimer (n = 2) und Trioxan über Kopf als Rückführstrom **11** abgetrennt werden. Der Sumpfabzugsstrom **12** der zweiten Destillationskolonne **10** wird einer dritten Kolonne **13** zugeführt, in der über Kopf ein Gemisch aus trimerem und tetramerem Polyoxymethylendialkylether (n = 3, 4) abgetrennt wird. Am Kolonnensumpf wird ein Rückführstrom **15** aus pentamerem Polyoxymethylendialkylether und höheren Polyoxymethylendialkylethern (n > 4) erhalten.

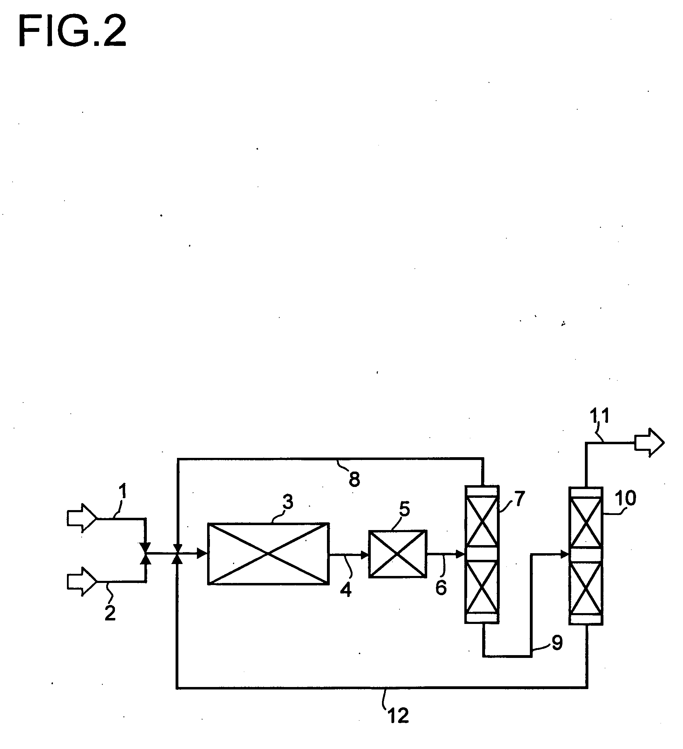

Figur 2 gibt das Verfahrensfließbild gemäß einer weiteren Ausführungsform des erfindungsgemäßen Verfahrens wieder. Im Unterschied zu dem in Figur 1 dargestellten Verfahren sind hier die erste und die zweite Destillationskolonne zu einer einzigen Destillationskolonne **7** zusammengefasst. Diese kann auch eine geteilte Kolonne sein, wie z.B. in US 2,471,134 beschrieben. Dementsprechend wird ein Rückführstrom **8** aus Dialkylether, Dimer (n=2) und Trioxan und ein Rückführstrom **12** aus pentamerem Polyoxymethylendialkylether und höheren Polyoxymethylendialkylethern (n > 4) erhalten. Der Sumpfabzugsstrom **9** der Destillationskolonne **7** wird einer zweiten Kolonne **10** zugeführt, in der über Kopf das Gemisch aus trimerem und tetramerem Polyoxymethylendialkylether (n = 3, 4) abgetrennt wird.

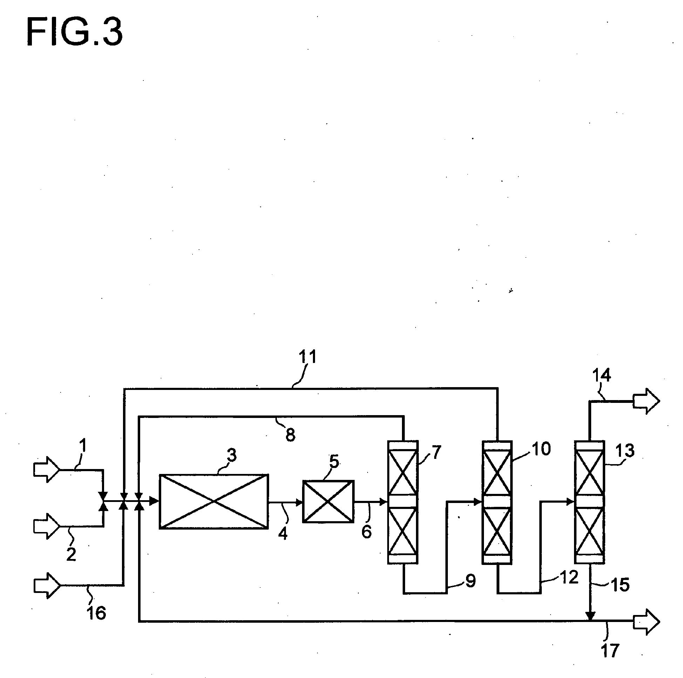

Figur 3 gibt das Verfahrensfließbild gemäß einer weiteren Ausführungsform des erfindungsgemäßen Verfahrens wieder. Im Unterschied zu dem Verfahren gemäß Figur 1 wird ein homogener Katalysator eingesetzt, welcher als weiterer Einspeisungsstrom **16** in den Reaktor **3** eingespeist wird. Auf ein dem Reaktor **3** nachgeschaltetes Bett aus anionischem Ionenaustauscherharz kann auch verzichtet werden und der Produktstrom **4** der Umsetzung direkt der ersten Destillationskolonne **7** zugeführt werden. Der Sumpfabzug **15** der dritten Destillationskolonne enthält zusätzlich den homogenen Katalysator. Ein kleiner Teilstrom **17** kann von dem Rückführstrom **15** abgetrennt und aus dem Verfahren ausgeschleust werden, wobei der Katatysatorverlust durch den Einsatzstrom **16** kompensiert wird.

### Beispiele

### Beispiel 1

30 g Trioxan und 63 g Dimethylether werden mit 0,2 g Schwefelsäure 16 Stunden lang bei 100 °C erhitzt. Nach 1, 2, 3, 4, 5, 6, 7, 8 und 16 Stunden wird jeweils eine Probe genommen und gaschromatographisch analysiert. Nach 8 Stunden wurde die Gleichgewichtszusammensetzung erreicht. DME entweicht beim Entspannen. Die Zusammensetzung war wie folgt gekennzeichnet: 18 % n = 2, 58 % n = 3, 16 % n = 4, Rest n > 4 und Probenahme / Analysenfehler.

### Beispiel 2

17 g Trioxan, 20 g DME und 15 g Ionenaustauscherharz Amberlite® IR 120 werden 24 Stunden bei 100 °C erhitzt. Nach 24 Stunden wird eine Probe genommen und gaschromatographisch analysiert. Das Gemisch enthält Polyoxymethylendimethylether in folgender Verteilung (in Gew.-%): 19 % n = 2, 64 % n = 3, 1 % n = 4, Rest n > 4.

## Patentansprüche

1. Verfahren zur Herstellung von Polyoxymethylendialkylethern der Formel
H₂ₘ₊₁CmO(CH₂O)ₙCₘH₂ₘ₊₁
mit n=2-10,
m, gleich oder verschieden, = 1 oder 2
bei dem ein Dialkylether, ausgewählt aus Dimethylether, Methylethylether und Diethylether, und Trioxan in einen Reaktor eingespeist werden und in Gegenwart eines sauren Katalysators umgesetzt werden, wobei die durch den Dialkylether, Trioxan und/oder den Katalysator in das Reaktionsgemisch eingebrachte Wassermenge < 1 Gew.-%, bezogen auf das Reaktionsgemisch, beträgt.

2. Verfahren nach Anspruch 1, **dadurch gekennzeichnet, dass** aus dem Gemisch der Umsetzung eine Fraktion enthaltend Polyoxymethylendialkylether mit n = 3 und 4 destillativ gewonnen wird und Dialkylether, Trioxan und Polyoxymethylendialkylether mit n < 3 und optional n > 4 in die Umsetzung zurückgeführt werden.

3. Verfahren nach Anspruch 2, **dadurch gekennzeichnet, dass** aus dem Gemisch der Umsetzung eine erste Fraktion enthaltend Dialkylether, eine zweite Fraktion enthaltend Polyoxymethylendialkylether mit n = 2 und Trioxan, eine dritte Fraktion enthaltend Polyoxymethylendialkylether mit n = 3 und 4 und eine vierte Fraktion enthaltend Polyoxymethylendialkylether mit n > 4 gewonnen werden.

4. Verfahren nach Anspruch 3, **dadurch gekennzeichnet, dass** die erste Fraktion in einer ersten Destillationskolonne von dem Gemisch der Umsetzung abgetrennt wird, aus dem verbleibenden Gemisch in einer zweiten Destillationskolonne die zweite Fraktion abgetrennt wird und das verbleibende Gemisch in einer dritten Destillationskolonne in die dritte und die vierte Fraktion aufgetrennt wird.

5. Verfahren nach Anspruch 3 oder 4, **dadurch gekennzeichnet, dass** die erste und die zweite Fraktion in die Umsetzung zurückgeführt werden.

6. Verfahren nach Anspruch 5, **dadurch gekennzeichnet, dass** die vierte Fraktion in die Umsetzung zurückgeführt wird.

7. Verfahren nach einem der Ansprüche 1 bis 6, **dadurch gekennzeichnet, dass** die erste Destillationskolonne bei einem Druck von 0,1 bis 100 bar, die zweite Destillationskolonne bei einem Druck von 0,05 bis 1 bar und die dritte Destillationskolonne bei einem Druck von 0,001 bis 0,5 bar betrieben wird.

8. Verfahren nach einem der Ansprüche 1 bis 7, **dadurch gekennzeichnet, dass** die in das Reaktionsgemisch eingebrachte Wassermenge < 0,2 Gew.-% beträgt.

9. Verfahren nach einem der Ansprüche 1 bis 8, **dadurch gekennzeichnet, dass** der saure Katalysator ein homogener oder heterogener Katalysator, ausgewählt aus Mineralsäuren, Sulfonsäuren, Heteropolysäuren, sauren Ionenaustauscherharzen, Zeolithen, Aluminosilikaten, Siliziumdioxid, Aluminiumoxid, Titandioxid und Zirkondioxid, ist.

10. Verfahren nach einem der Ansprüche 1 bis 9, **dadurch gekennzeichnet, dass** die Umsetzung bei einem Druck von 1 bis 200 bar und einer Temperatur von minus 20 bis +200 °C durchgeführt wird.

11. Verfahren nach einem der Ansprüche 1 bis 10, **dadurch gekennzeichnet, dass** als Dialkylether Dimethylether eingesetzt wird.

## Claims

1. A process for preparing polyoxymethylene dialkyl ethers of the formula
H₂ₘ₊₁CₘO(CH₂O)ₙCₘH₂ₘ₊₁
where n = 2 - 10,
m, identically or differently, = 1 or 2,
in which a dialkyl ether selected from dimethyl ether, methyl ethyl ether and diethyl ether, and trioxane are fed into a reactor and reacted in the presence of an acidic catalyst, the amount of water introduced into the reaction mixture by the dialkyl ether, trioxane and/or the catalyst being < 1% by weight based on the reaction mixture.

2. The process according to claim 1, wherein a fraction comprising polyoxymethylene dialkyl ether where n = 3 and 4 is obtained by distillation from the reaction mixture, and dialkyl ether, trioxane and polyoxymethylene dialkyl ether where n < 3 and optionally n > 4 are recycled into the reaction.

3. The process according to claim 2, wherein a first fraction comprising dialkyl ether, a second fraction comprising polyoxymethylene dialkyl ether where n = 2 and trioxane, a third fraction comprising polyoxymethylene dialkyl ether where n = 3 and 4, and a fourth fraction comprising polyoxymethylene dialkyl ether where n > 4 are obtained from the reaction mixture.

4. The process according to claim 3, wherein the first fraction is removed from the reaction mixture in a first distillation column, the second fraction is removed from the remaining mixture in a second distillation column and the remaining mixture is separated into the third and the fourth fraction in a third distillation column.

5. The process according to claim 3 or 4, wherein the first and the second fraction are recycled into the reaction.

6. The process according to claim 5, wherein the fourth fraction is recycled into the reaction.

7. The process according to any of claims 1 to 6, wherein the first distillation column is operated at a pressure of from 0.1 to 100 bar, the second distillation column at a pressure of from 0.05 to 1 bar and the third distillation column at a pressure of from 0.001 to 0.5 bar.

8. The process according to any of claims 1 to 7, wherein the amount of water introduced into the reaction mixture is < 0.2% by weight.

9. The process according to any of claims 1 to 8, wherein the acidic catalyst is a homogeneous or heterogeneous catalyst selected from mineral acids, sulfonic acids, heteropolyacids, acidic ion exchange resins, zeolites, aluminosilicates, silicon dioxide, aluminum oxide, titanium dioxide and zirconium dioxide.

10. The process according to any of claims 1 to 9, wherein the reaction is carried out at a pressure of from 1 to 200 bar and a temperature of from minus 20 to +200°C.

11. The process according to any of claims 1 to 10, wherein the dialkyl ether used is dimethyl ether.

## Revendications

1. Procédé pour la préparation de polyoxyméthylènedialkyléthers de formule
H₂ₘ₊₁CₘO(CH₂O)ₙCₘH₂ₘ₊₁
avec n = 2-10,
m, identique ou différent, = 1 ou 2
dans lequel un dialkyléther, choisi parmi le diméthyléther, le méthyléthyléther et le diéthyléther, et du trioxane sont injectés dans un réacteur et transformés en présence d'un catalyseur acide, la quantité d'eau introduite par le dialkyléther, le trioxane et/ou le catalyseur dans le mélange réactionnel étant < 1% en poids, par rapport au mélange réactionnel.

2. Procédé selon la revendication 1, **caractérisé en ce qu'**on extrait par distillation du mélange de la transformation une fraction contenant du polyoxyméthylènedialkyléther avec n = 3 et 4 et le dialkyléther, le trioxane et le polyoxyméthylènedialkyléther avec n < 3 et éventuellement n > 4 sont recyclés dans la transformation.

3. Procédé selon la revendication 2, **caractérisé en ce qu'**on extrait du mélange de la transformation une première fraction contenant du dialkyléther, une deuxième fraction contenant du polyoxyméthylènedialkyléther avec n = 2 et du trioxane, une troisième fraction contenant du polyoxyméthylènedialkyléther avec n = 3 et 4 et une quatrième fraction contenant du polyoxyméthylènedialkyléther avec n > 4.

4. Procédé selon la revendication 3, **caractérisé en ce que** la première fraction est séparée dans une première colonne de distillation du mélange de la transformation, on sépare du mélange résiduel dans une deuxième colonne de distillation la deuxième fraction et on sépare le mélange résiduel dans une troisième colonne de distillation en une troisième et une quatrième fraction.

5. Procédé selon la revendication 3 ou 4, **caractérisé en ce que** la première et la deuxième fraction sont recyclées dans la transformation.

6. Procédé selon la revendication 5, **caractérisé en ce que** la quatrième fraction est recyclée dans la transformation.

7. Procédé selon l'une quelconque des revendications 1 à 6, **caractérisé en ce que** la première colonne de distillation est exploitée à une pression de 0,1 à 100 bars, la deuxième colonne de distillation à une pression de 0,05 à 1 bar et la troisième colonne de distillation à une pression de 0,001 à 0,5 bar.

8. Procédé selon l'une quelconque des revendications 1 à 7, **caractérisé en ce que** la quantité d'eau introduite dans le mélange réactionnel est < 0,2% en poids.

9. Procédé selon l'une quelconque des revendications 1 à 8, **caractérisé en ce que** le catalyseur acide est un catalyseur homogène ou hétérogène choisi parmi les acides minéraux, les acides sulfoniques, les hétéropolyacides les résines acides échangeuses d'ions, les zéolithes, les aluminosilicates, le dioxyde de silicium, l'oxyde d'aluminium, le dioxyde de titane et le dioxyde de zirconium.

10. Procédé selon l'une quelconque des revendications 1 à 9, **caractérisé en ce que** la transformation est réalisée à une pression de 1 à 200 bars et une température de moins 20 à +200°C.

11. Procédé selon l'une quelconque des revendications 1 à 10, **caractérisé en ce qu'**on utilise, comme dialkyléther, du diméthyléther.
